Europäisches Patentamt

European Patent Office

Office européen des brevets

(11) Veröffentlichungsnummer: **0 098 483**
**B1**

(12) **EUROPÄISCHE PATENTSCHRIFT**

(45) Veröffentlichungstag der Patentschrift:
**12.03.86**

(21) Anmeldenummer: **83106184.1**

(22) Anmeldetag: **24.06.83**

(51) Int. Cl.⁴: **C 07 D 251/32** // C09D5/08

(54) Verfahren zur Herstellung von Blei- und Zinkcyanuraten.

(30) Priorität: **02.07.82 DE 3224766**

(43) Veröffentlichungstag der Anmeldung:
**18.01.84 Patentblatt 84/3**

(45) Bekanntmachung des Hinweises auf die Patenterteilung:
**12.03.86 Patentblatt 86/11**

(84) Benannte Vertragsstaaten:
**DE FR GB IT NL**

(56) Entgegenhaltungen:
**DE - B - 2 807 698**

**CHEMICAL ABSTRACTS, vol. 89, nr. 3, 17. Juli 1978, Columbus, Ohio, USA, V.I. ZAGRANICHNYI "Preparation of lead cyanurate by a "dry" method, Seite 648, Zusammenfassung nr. 24261z
CHEMICAL ABSTRACTS, vol. 91, nr. 9, 27. August 1979, Columbus, Ohio, USA, O.S. RUKEVICH et al. "Preparation of monosubstituted salts of cyanuric acid", Seite 596, Zusammenfassung nr. 74572p**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Harth, Hubert, Dr., Buchenstrasse 31, D-4000 Düsseldorf 13 (DE)**
Erfinder: **Witthaus, Martin, Dr., Burgmüller Strasse 7, D-4000 Düsseldorf (DE)**
Erfinder: **Gress, Wolfgang, Westfalenweg 247, D-5600 Wuppertal 1 (DE)**

ACTORUM AG

## Beschreibung

Die Erfindung betrifft ein Verfahren zur Herstellung von Blei- und Zinkcyanuraten aus PbO bzw. ZnO und Cyanursäure, wobei die Umsetzung der entsprechenden Oxide mit der Cyanursäure in wässeriger Paste ohne Zusatz von Katalysatoren vorgenommen wird.

Nach der DE-AS Nr. 2807698 dienen Blei- und Zinkcyanurate als Korrosionsschutzüberzugsmittel für Metalloberflächen. Sie können nach dieser Erfindung hergestellt werden, indem Blei- bzw. Zinkoxid und Cyanursäure in kochendem Wasser suspendiert werden, wobei zur Beschleunigung der Reaktion als Katalysator Essigsäure zugesetzt werden muss. Die Cyanurate fallen aus der wässerigen Lösung aus und müssen abfiltriert, gewaschen und getrocknet werden.

Nachteilig an diesem Verfahren ist zum einen, dass diese Suspensionen, wenn sie noch rührfähig sein sollen, nur einen geringen Feststoffgehalt von maximal 14% beim Zinkcyanurat bzw. 20% beim Bleicyanurat haben. Dies schlägt sich in hohen Fertigungskosten nieder. Zum anderen müssen die Salze für ihren Einsatz als Korrosionsschutzüberzugsmittel frei von Fremdionen sein, weshalb eine aufwendige Waschprozedur zur Entfernung der als Katalysator zugesetzten Essigsäure erforderlich ist.

In „Chemical Abstracts", Bd. 89, Nr. 3 (17.7.78), 24261 z, wird ein Verfahren zur Herstellung von Bleicyanurat $[Pb(H_2O_3C_3N_3)]_2$ aus PbO und Cyanursäure beschrieben, bei dem die Reaktionskomponenten in trockener Form bei 100 bis 110° C und einer Dauer von 90 min miteinander vermischt werden. Es wird ein Umsetzungsgrad von >90% erzielt.

Daher stellt sich die Erfindung die Aufgabe, ein verbessertes Verfahren zu schaffen, bei dem die genannten Nachteile nicht auftreten.

Überraschenderweise wurde nun gefunden, dass die Blei- und Zinkcyanurate auch hergestellt werden können, indem man zu einer Mischung der entsprechenden Oxide und der Cyanursäure nur so viel Wasser zusetzt, dass eine knetbare Paste entsteht, und diese Paste der Scherwirkung von Misch-, Dispergier- und Kneteinrichtungen aussetzt. Ein Zusatz von Katalysatoren ist dann nicht erforderlich und die Reaktion ist schneller als beim oben geschilderten Suspensionsverfahren beendet.

Dementsprechend betrifft die Erfindung ein Verfahren zur Herstellung von Blei- und Zinkcyanuraten durch Umsetzung von PbO bzw. ZnO und Cyanursäure in Gegenwart von Wasser, das dadurch gekennzeichnet ist, dass man PbO oder ZnO und Cyanursäure zu einer knetbaren Paste mit einem möglichst geringen Wassergehalt von 10 bis 80 Gew.-% — bezogen auf die Paste — vermischt und die erhaltene Paste der Scherwirkung unterwirft, wobei auf 50 bis 250° C erhitzt wird.

Der Wassergehalt der Paste sollte möglichst gering sein und ist von der Art des herzustellenden Salzes abhängig. Auf jeden Fall muss so viel Wasser zugesetzt werden, dass eine knetbare Paste entsteht. Der Wassergehalt liegt zwischen 10 und 80 Gew.-% — bezogen auf die Paste.

Der bevorzugte Temperaturbereich für die Umsetzung liegt bei 100 bis 180° C. Der Druck beträgt 1 bis 10 bar.

Für die erfindungsgemässe Herstellung der Blei- und Zinkcyanurate sind alle Apparaturen geeignet, die eine ausreichende Scherung erzeugen, wie beispielsweise Behälter mit Rotor-Stator-Maschinen, Mischer und Kneter.

Besonders gut geeignet sind solche Apparaturen, in denen die Salze im Anschluss an die Umsetzung getrocknet werden können. Ein Beispiel hierfür sind Schaufeltrockner mit eingebauten Dispergiereinrichtungen wie z. B. Stift- oder Messermühlen.

Da die Cyanursäure bekanntlich eine dreibasische Säure ist, umfasst die Erfindung die Herstellung sämtlicher, d. h. der neutralen, sauren und basischen Salze des zweiwertigen Bleis und Zinks.

Das erfindungsgemässe Verfahren hat den Vorteil, dass bei der Herstellung der Blei- und Zinkcyanurate kein Katalysator verwendet wird, so dass eine aufwendige Prozedur zur Entfernung des Katalysators nicht erforderlich ist. Ferner können die Salze in fester Form erhalten werden, so dass die Nachteile, die sich beim bekannten Verfahren aufgrund des geringen Feststoffgehalts ergeben, nicht vorhanden sind.

Die Erfindung wird anhand der Beispiele weiter erläutert.

*Beispiel 1:*

In einem Schaufeltrockner mit eingebauter Stiftmühle wurden 73,3 kg entsalztes Wasser, 120 kg Bleioxid (PbO) und 46,7 kg Cyanursäure miteinander vermischt. Anschliessend wurde auf 105 bis 110° C aufgeheizt, wobei der Druck auf 1,6 bar anstieg. Nach 2,5 h war die Umsetzung vollständig beendet. Das entstandene Bleicyanurat wurde anschliessend in demselben Apparat bei 40 mbar auf eine Restfeuchte kleiner als 1% getrocknet. Es wurden 163,2 kg neutrales Bleicyanurat $[Pb_3(C_3N_3O_3)_2 \times 2H_2O]$ erhalten.

*Beispiel 2:*

In einem Schaufeltrockner mit eingebauter Messermühle wurden 84 kg entsalztes Wasser, 22 kg Zinkoxid (ZnO) und 14,3 kg Cyanursäure miteinander vermischt. Anschliessend wurde auf 105 bis 110° C aufgeheizt, wobei der Druck auf 2,1 bar anstieg. Nach 2 h war die Reaktion vollständig beendet. Das entstandene basische Zinkcyanurat wurde in demselben Apparat bei 40 mbar auf eine Restfeuchte kleiner als 1% getrocknet. Es wurden 26,1 kg basisches Zinkcyanurat $[Zn_3(C_3N_3O_3)_2 \times 2ZnO \times 3H_2O]$ erhalten.

*Beispiel 3:*

In einem Schaufeltrockner mit eingebauter Messermühle wurden 50 kg entsalztes Wasser, 80 kg Bleioxid (PbO) und 31,1 kg Cyanursäure miteinander vermischt. Anschliessend wurde auf 140 bis 150° C aufgeheizt. Nach 1 h war die Umsetzung vollständig beendet. Das entstandene Bleicyanurat wurde anschliessend in demselben Apparat bei 40 mbar auf eine Restfeuchte kleiner als 1% getrocknet. Es wurden 108,1 kg neutrales Bleicyanurat $[Pb_3(C_3N_3O_3)_2 \times 2H_2O]$ erhalten.

*Beispiel 4:*

In einem Schaufeltrockner mit eingebauter Messermühle wurden 85 kg entsalztes Wasser, 22,3 kg Zinkoxid (ZnO) und 14,5 kg Cyanursäure miteinander vermischt. Anschliessend wurde auf 140 bis 150° C aufgeheizt. Nach 1 h war die Reaktion vollständig beendet. Das entstandene basische Zinkcyanurat wurde in demselben Apparat bei 40 mbar auf eine Restfeuchte kleiner als 1% getrocknet. Es wurden 36,3 kg basisches Zinkcyanurat $[Zn_3(C_3N_3O_3)_2 \times 2ZnO \times 3H_2O]$ erhalten.

## Patentansprüche

1. Verfahren zur Herstellung von Blei- und Zinkcyanuraten durch Umsetzung von PbO bzw. ZnO und Cyanursäure in Gegenwart von Wasser, dadurch gekennzeichnet, dass man PbO oder ZnO und Cyanursäure zu einer knetbaren Paste mit einem möglichst geringen Wassergehalt von 10 bis 80 Gew.-% — bezogen auf die Paste — vermischt und die erhaltene Paste der Scherwirkung unterwirft, wobei auf 50 bis 250° C erhitzt wird.

2. Verfahren nach Anspruch 1, dadurch gekennzeichnet, dass die Umsetzung bei Temperaturen von 100 bis 180° C erfolgt.

3. Verfahren nach den Ansprüchen 1 und 2, dadurch gekennzeichnet, dass die Scherwirkung durch Umsetzung in Mischern, Dispergierapparaturen oder Knetern erfolgt.

4. Verfahren nach den Ansprüchen 1 bis 3, dadurch gekennzeichnet, dass die Mischung und Umsetzung in einem Schaufeltrockner mit eingebauter Stiftmühle oder Messermühle erfolgt.

## Claims

1. A process for the production of lead and zinc cyanurates by reaction of PbO or ZnO and cyanuric acid in the presence of water, characterized in that PbO or ZnO and cyanuric acid are mixed to form a kneadable paste having a very low water content of from 10 to 80% by weight, based on the paste, and the paste obtained is subjected to shearing, being heated to 50-250° C at the same time.

2. A process as claimed in Claim 1, characterized in that the reaction is carried out at temperatures of from 100 to 180° C.

3. A process as claimed in Claims 1 and 2, characterized in that shearing is carried out by reaction in mixers, dispersers or kneaders.

4. A process as claimed in Claims 1 to 3, characterized in that mixing and reaction are carried out in a trough dryer with a built-in disc attrition mill or cross-beater mill.

## Revendications

1. Procédé pour la fabrication de cyanurates de plomb et de zinc, par réaction de PbO ou de ZnO et d'acide cyanurique en présence d'eau, caractérisé en ce que le PbO ou le ZnO et l'acide cyanurique sont mélangés en une pâte triturable, avec une teneur en eau la plus faible possible de 10 à 80% en poids calculé sur la pâte, et que la pâte obtenue est soumise à une action de cisaillement, tout en chauffant de 50 à 250° C.

2. Procédé suivant la revendication 1, caractérisé en ce que la réaction est effectuée à une température de 100 à 180° C.

3. Procédé suivant les revendications 1 et 2, caractérisé en ce que l'action de cisaillement s'opère en faisant réagir dans des mélangeurs, des appareils de dispersion où des machines à pétrir.

4. Procédé suivant les revendications 1 à 3, caractérisé en ce que le mélange et la réaction s'opèrent dans un séchoir à pelles, avec broyeur à aiguilles ou à lames incorporé.